Europäisches Patentamt

European Patent Office

Office européen des brevets

(11) Publication number: **0 244 873 B1**

# EUROPEAN PATENT SPECIFICATION

(45) Date of publication of patent specification: **05.08.92**

(51) Int. Cl.⁵: **C12M 3/00**, C12M 1/16, C12M 1/00

(21) Application number: **87106710.4**

(22) Date of filing: **08.05.87**

---

(54) **Cell culture method.**

---

(30) Priority: **09.05.86 JP 106393/86**

(43) Date of publication of application:
**11.11.87 Bulletin 87/46**

(45) Publication of the grant of the patent:
**05.08.92 Bulletin 92/32**

(84) Designated Contracting States:
**CH DE FR LI**

(56) References cited:
**FR-A- 948 111**

**CHEMICAL ABSTRACTS, vol. 83, no. 15, 13th October 1975, page 376, abstract no. 130029r, Columbus, Ohio, US; & JP-A-75 09 868 (YAMASA SHOYU CO., LTD) 16-04-1975**

**BIOTECHNOLOGY & BIOENGINEERING, vol. 26, no. 9, September 1984, pages 1098-1107, John Wiley & Sons, Inc, New York, US; W.R. GIBBONS et al.: "A continuous, farm-scale, solid-phase fermentation process for fuel ethanol and protein feed production from fodder beets",**

(73) Proprietor: **RIKAGAKU KENKYUSHO**
**2-1 Hirosawa**
**Wako-shi Saitama-ken(JP)**

Proprietor: **LION CORPORATION**
**3-7, Honjo 1-chome**
**Sumida-ku Tokyo(JP)**

Proprietor: **SHINKO PANTEC CO., LTD.**
**4-78, Wakihama-cho 1-chome Chuo-ku Kobe-shi, Hyogo-ken(JP)**

(72) Inventor: **Endo, Isao**
**5-7-6, Honda**
**Kokubunji-shi Tokyo(JP)**
Inventor: **Nagamune, Teruyuki**
**2nd-Chuo-manshion 404 2-16-14, Chuo Kamifukuoka-shi Saitama-ken(JP)**
Inventor: **Nishimura, Minoru**
**2-2-6-305, Izumi-cho**
**Narashino-shi Chiba-ken(JP)**
Inventor: **Kobayashi, Tetsuo**
**2-9-7, Matsugae-cho Kita-ku**
**Kobe-shi Hyogo-ken(JP)**

(74) Representative: **Vossius & Partner**
**Siebertstrasse 4 P.O. Box 86 07 67**
**W-8000 München 86(DE)**

**Description**

This invention relates to a cell culture method for proliferating microorganisms, particularly fungi such as molds, actinomycetes, animal cells, plant cells and so on to produce useful substances such as antibiotics, enzymes, proteins, polysaccharides, physiologically active substances, and animal and plant hormones.

In culture of fungi, a submerged culture method or a solid-state culture method is selectively used, depending on the kind of cells and the objective product.

W.R. Gibbons et al describe in Biotechnology and Bioengineering 26 (1984), pages 1098-1107, a continuous fermentation process using an auger as a solid-phase fermentor.

In Chemical Abstracts, Vol. 83, 1975, 130029r, T. Fujishima et al describe a process for culturing fungi using urethane sponge as solid-phase.

FR-A-948 111 relates to a fermentation process utilizing mycelium fungi which are immersed into an aqueous solution before said process starts and wherein the mycelium is supported by a rough solid like chamotte or limestone.

In the submerged culture method, pellet growth or pulpy growth occurs, depending on the kind of cells and culture condition. In the solid-state culture method, a natural substance such as rice or bran is used as medium, and culture is carried out in standing state. Recently, instead of the solid-state culture method in which a natural substance is used as a medium, a method in which a liquid medium is impregnated into sponge-like substance and culture is carried out in a standing state has been reported.

In culture of animal and plant cells, the submerged culture method and the solid-state culture method are also used. In the submerged culture method, a cell suspended culture method in which cells are directly suspended in the liquid medium, and another cell suspended culture method in which cells are encapsulated with or attached to carriers such as microcapsules or high-polymer resin pieces and suspended with the carriers are used. In the solid-state culture method, a method of attaching cells to the surface of a medium such as an agar medium and cultivating them in standing state is used.

As described above, in culture of fungi, the submerged culture method and the solid-state culture method have been conventionally used. The submerged culture method causes distinct problems, depending on the morphology of cellular growth as follows. In the pellet growth, according as the diameter of pellets becomes large, the mass transfer of oxygen and nutritive substances becomes the rate-controlling step. As a result, the cell growth rate decreases extremely and productivity of metabolites decreases. On the other hand, in the pulpy growth, the viscosity of the culture medium increases extremely, the medium is not mixed uniformly, and very high agitation power is needed. Furthermore, separation of the objective product from the medium including cells becomes difficult.

In the solid-state culture method, since the composition of solid natural medium varies in each lot, the amount of cellular growth and the yield of objective product varies greatly. And further since the objective product is stored in the solid medium, the separation of the objective product and its purification are difficult.

In the standing culture method of impregnating a spongy material with a liquid medium, since the objective product is stored in the liquid media, separation and purification of the objective product are easy. However, since contact of the medium with air is not sufficient and mass transfer is mainly caused by diffusion, cells grow nonuniformly in spongy material and growth rate decreases. Consequently, the productivity of the objective product is suppressed.

The same problems as in the case of microorganisms such as fungi also arise in culture of animal and plant cells.

The above described problems are solved by this cell culture method having a step of moving a porous material including a liquid medium and the cells to be proliferated, thereby supplying air to the surface of the porous material.

Thus the process of the invention relates to a cell culture method comprising a step of moving porous material which contains a liquid medium in an amount of from 15 cm$^3$ to 60 cm$^3$ impregnated per 100 cm$^3$ of the porous material and the cells to be proliferated, wherein the moving of the porous material is conducted by shaking, vibration, mixing or a combination of these.

According to this method, the contact of the cells with the liquid medium and air (oxygen) becomes easy, a uniform biological film is formed on the surface of the porous material, the cell growth rate increases, enzyme intracellular activity is enhanced, extracellular secretion is accelerated, and productivity of the objective product increases.

As the porous material may be used a formed synthetic high polymer such as urethane foam, natural sponge, or synthetic or natural fiber finished in one, two or three dimensions, in other words, finished to be filamentous, lace-like, fabric-like or plush.

With regard to the shape of the porous material, a sphere is preferable in view of its large ratio of surface to volume. However, a cube, rectangular parallelopiped or any other shape may be used so far as it does not obstruct the movement of the porous material.

The moving of the porous material is achieved by shaking, vibration, mixing or a combination of these, in other words, up and down motion, to and fro motion, rotary motion, or a motion combining these may be used. The effect of this invention is enhanced if the porous material is constantly moved.

The amount of the liquid medium with which the porous material is impregnated is $15cm^3$ to $60cm^3$ per $100cm^3$ of porous material. However, the amount varies, depending on the characteristics of the porous material such as its specific gravity and porosity, growth rate of used cells, production rate of the objective product and so on. If the impregnation ratio is increased so that the liquid medium covers the whole surface of the porous material, mass transfer of oxygen to the cells is prevented. On the other hand, if the impregnation ratio is extremely low, supply of the liquid medium to the cells becomes insufficient and nonuniform, and it becomes difficult to recover the objective product.

In this invention, since the mass transfer is not the rate-controlling step, cellular growth rate and intracellular activity of enzymes increase, extracellular secretion is enhanced, and the productivity of a useful objective product such as an antibiotic ( streptomycin, cephamycin, oxytetracycline, erythoromycin, kanamycin, etc. ), enzyme (amylase, protease, pectinase, cellulase, lipase, glucoamylase, glucoisomerase, etc. ), protein ( single cell protein, etc. ), polysaccharide ( arabinose, mannose, rhamnose, dextran, etc. ), physiological active substance (vitamin $B_{12}$, vitamin $B_2$, vitamin C, etc. ), or animal or plant hormone ( auxin, cytokinin, gibberellin, steroid hormone, insulin, lymphokine, etc. ) is enhanced, and mass production and direct recovery of highly concentrated objective product from culture solution becomes possible.

Fig. 1 is a graph showing the relationship between the saccharification activity of the strain JMC2058 and the porous material size,

Fig. 2 is a graph showing the relationship between the saccharification activity of the strain JMC2058 and the impregnation ratio of the liquid medium,

Fig. 3 is a schematic diagram of a cell culture system for carrying out this invention on industrial level, and

Fig. 4 is a detail sectional view of a cell culture apparatus and a conical shape press used in the cell culture system of Fig. 3.

Hereinbelow, the invention will be described in detail by way of examples.

Example 1: Effect of moving porous material

Pieces of cubic porous material about 5mm in side length of urethane foam, 2.5g in total weight ($51.5cm^3$ in total volume) were put into a conical flask. The porous material was impregnated with 30ml Czapek medium consisting of 30g saccharose, 5g yeast extract, 1g $K_2HPO_4$, 3g $NaNO_3$, 0.5g $MgSO_4 \cdot 7H_2O$, 0.5g KCl, and 0.01g $FeSO_4 \cdot 7H_2O$, with one liter distilled water. Next, Aspergillus oryzae (JCM2239) was inoculated into the flask. Culture was carried out in two ways:by standing culture in an incubator at 24°C and by shaking culture at 200rpm shaking rotational speed at 24°C.

At the sixth day from the beginning of the culture, the urethane foam was taken out, the liquid medium was wrung out and saccharification activity ($\alpha$-amylase activity) of the liquid medium was measured by the Blue-Value method. The same experiment was carried out for Aspergillus oryzae var. brunneus(JCM2058). The results are shown in Table 1.

TABLE 1

| Saccharification activity ($\alpha$-amylase activity) | | |
|---|---|---|
| Culture method | JCM2239 | JCM2058 |
| Standing culture | 570 | 5465 |
| Shaking culture | 2230 | 7038 |

wherein $\alpha$-amylase activities are shown in

$$\left( DB \begin{array}{c} 40°C30' \\ mgA \end{array} \right),$$

which is the amount of amylose in milligram units separated in 30 minutes at 40°C.

For both kinds of cells, saccharification activities in the shaking culture of cells were larger than those in the standing culture. In the standing culture, pieces of porous material were combined with each other by mycelium, growth of cells on the surface of the porous material was not uniform, and medium and oxygen did not contact the cells well. On the other hand, when the porous material was moved by shaking, oxygen and medium contacted the cells well, and the biological film was uniformly formed on the whole surface of the porous material.

Example 2: Effects of size of porous material and impregnation ratio of liquid medium.

Pieces of cubic porous material of urethane foam 2.5g in total weight (51.5cm in total volume) of side lengths about 20mm, 10mm, 5mm and 2.5mm were put into respective 500ml flasks.The same Czapek medium as in Example 1 was poured in an amount of 46ml, 31ml, 15ml, 10ml, 8ml and 2.5ml into the respective flasks to prepare 90%, 60%, 30%, 20%, 15% and 5% impregnation ratios for each size. Next, Aspergillus oryzae var. brunneus(JCM2058) was inoculated into the flasks. Shaking culture was carried out at 200rpm shaking rotational speed at 24°C. At the sixth day from the beginning of the culture, the urethane foam was taken out and the liquid medium was wrung out and saccharification activities were measured. The results are shown in Table 2.

TABLE 2

| Saccharification activity ($\alpha$-amylase activity) of JCM2058 | | | | | | |
|---|---|---|---|---|---|---|
| Size (length) | Impregnation ratio | | | | | |
| | 90% | 60% | 30% | 20% | 15% | 5% |
| 20mm | 11259 | 13136 | 19502 | 21294 | 21841 | 6230 |
| 10mm | 12640 | 7407 | 13827 | 25441 | 22394 | 7146 |
| 5mm | 3628 | 7038 | 9172 | 14730 | 15399 | 4462 |
| 2.5mm | 6427 | 8721 | 10535 | 18413 | 19398 | 2603 |

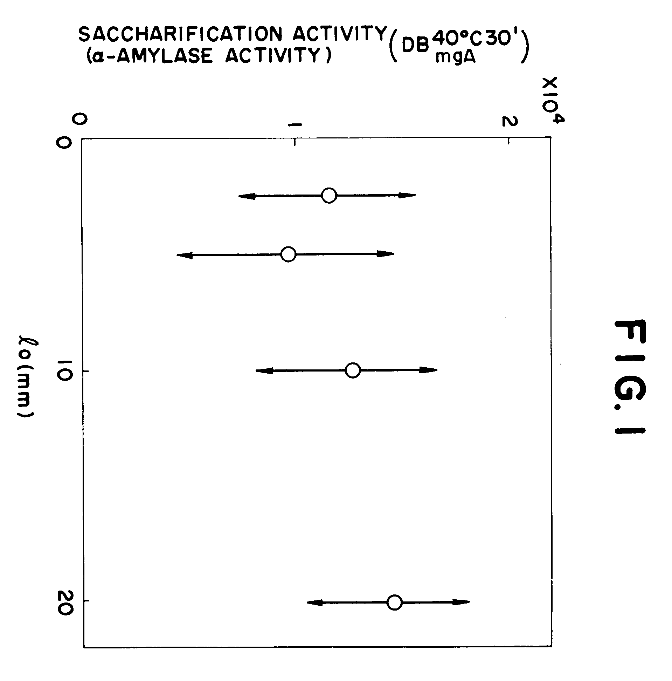

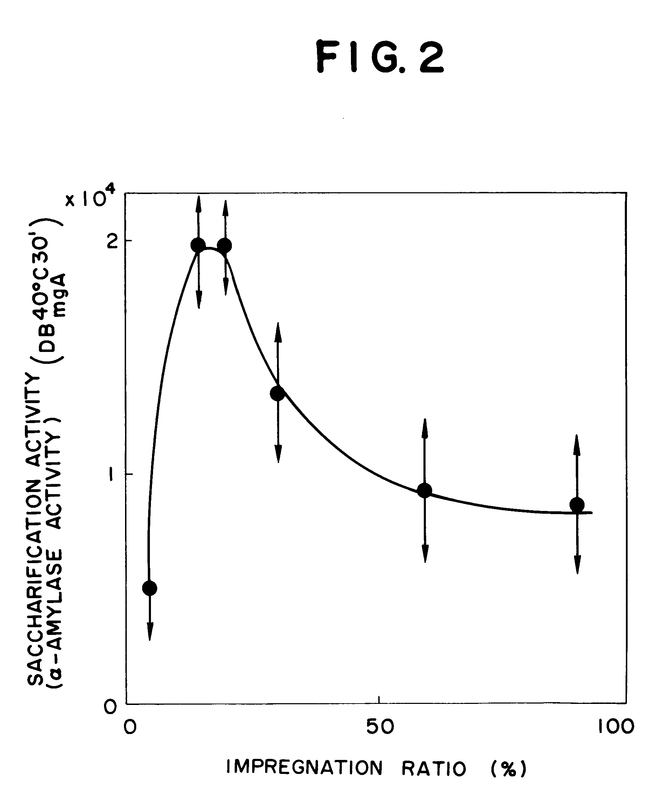

Fig. 1 shows the effect of porous material size on saccharification activity and Fig. 2 shows the effect of liquid medium impregnation ratio on saccharification activity.

In this example, no effect of porous material size on saccharification activity was observed. With regard to the impregnation ratio, a smaller ratio tends to increase the saccharification activity.However, too small a ratio slightly reduces the activity. This may be because when the impregnation ratio is too small, supply of medium to the biological film is not sufficient and growth of cells is not uniform in porous material. It can be found from Fig. 2 that from the point of yield an impregnation ratio of less than 60% is preferable while from the point of easy recovery of the objective product an impregnation ratio of more than 15% is preferable.

Example 3: Effect of rotary mixing of porous material by use of rotary drum

Pieces of cubic porous material of urethane foam about 10mm in side length, 120g in total weight (3500cm$^3$) were put into a rotary drum 25cm in diameter and 35cm in depth.As in Example 1, the porous material was impregnated with 1000ml Czapek medium. Aspergillus oryzae var. brunneus(JCM2085) was inoculated, and culture was carried out at 24°C, 60rpm rotational speed, and 0.5 l/min air flow. At the sixth day from the beginning of the culture, the urethane foam was taken out, the liquid medium was wrung out, and the saccharification activity ($\alpha$-amylase activity) was measured.

Two controls were prepared to be compared with the example.The same pieces of urethane foam 2.3g in weight (48cm$^3$ in volume) were put into 300ml conical flasks, respectively. In the control 1, the urethane foam was impregnated with 30ml Czapek medium. Culture was carried out by the standing culture method at 24°C. In the control 2, 100ml Czapek medium was poured into the 300ml conical flask. Culture was carried out by the shaking culture method at 24°C and a shaking rotational speed of 200rpm. Saccharification activities for controls 1 and 2 at the sixth day from the beginning of the culture were measured.

The results are shown in Table 3.

TABLE 3

| Saccharification activity ($\alpha$-amylase activity) of JCM2058 | |
| --- | --- |
| Culture method | Saccharification activity |
| rotary drum type culture | 12116 |
| standing culture (control 1) | 5465 |
| submerged culture (control 2) | 4403 |

In the standing culture method (control 1), as in example 1, growth of cells was not uniform. In the submerged culture method (control 2), cells grew pulpylike,but pellets about 5mm in diameter were included in the liquid medium, and the viscosity of the liquid medium was high during culture. On the other hand, in the culture method using a rotary drum, the pieces of porous material were rotated and mixed, so they did not always contact each other, and a uniform biological film of cells was formed. As shown in Table 3, the saccharification activity ($\alpha$-amylase activity) in Example 3 was two or three times as large as those in the standing culture method and the submerged culture method.

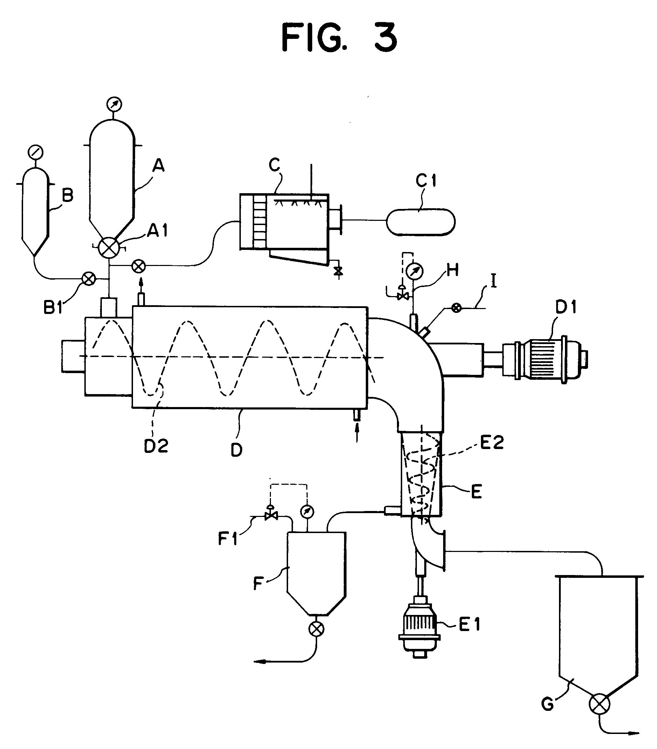

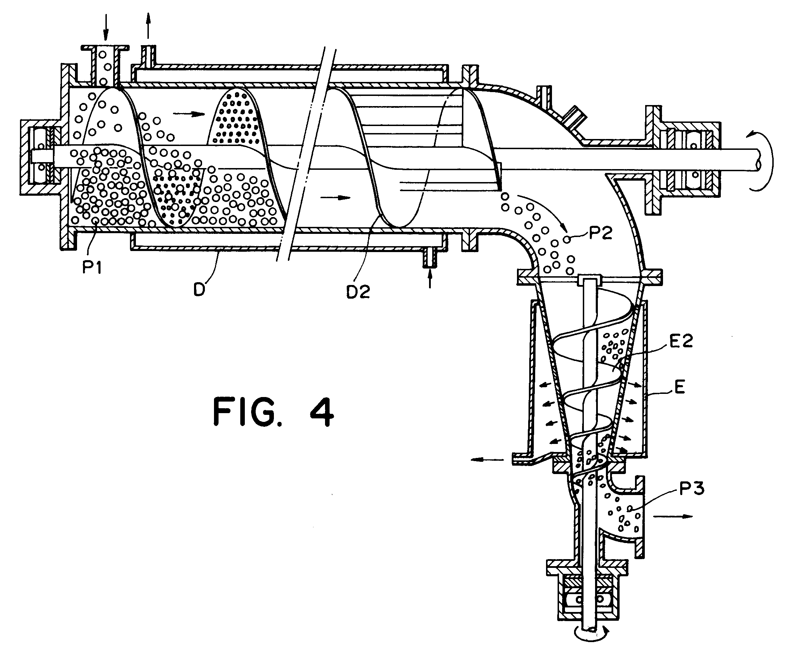

Referring to Figs. 3 and 4, there is shown a cell culture system for carrying out this invention on industrial level. A predetermined amount of porous material including liquid medium aseptically is supplied continually or batchwise from a porous material supply tank A through a rotary supply valve A1 into a cell culture apparatus body D. Cells are simultaneously supplied from a cell supply tank B through a supply valve B1 into the cell culture apparatus body D, and the supplied cells are inoculated on the porous material. Temperature and moisture are controlled and pressurized air is continually supplied from a fan C1 through an air-conditioner C. The pieces of porous material P1 including the cells and liquid medium are kneaded and mixed by a screw kneader blade D2 which is driven by a variable speed electric motor D1, and cell culture is accelerated.After proliferation of cells or production of an objective product finishes, the pieces of porous material P2 are pressed in a conical shape press E by a screw press blade E2 which is driven by a variable speed elecric motor E1, and the liquid medium is separated from cells. The separated liquid medium and the liquid removed and cell including porous material P3 are recovered in a liquid medium recover tank F and a porous material recover tank G, respectively. A pressure control exhaust nozzle H having a pressure control valve is disposed on an outlet of the cell culture apparatus body D, and another pressure control exhaust nozzle F1 is disposed on the conical style press E, whereby pressures in the cell culture apparatus body D and the conical style press E are maintained higher than that out of those apparatus. In the case that the objective product is an intracellular product, a sterile cleaning fluid is injected by a sterile cleaning fluid supply pipe I, cells are cleaned and recoverd in the porous material recover tank G together with the porous material after the porous material is pressed and dehydrated.

## Claims

1. A cell culture method comprising a step of moving porous material which contains a liquid medium in an amount of from 15cm$^3$ to 60cm$^3$ impregnated per 100cm$^3$ of the porous material and the cells to be proliferated, wherein the moving of the porous material is conducted by shaking, vibration mixing or a combination of these.

## Revendications

1. Procédé de culture cellulaire comprenant une étape de remuage d'un matériau poreux contenant un milieu liquide d'imprégnation en une quantité de 15 cm$^3$ à 60 cm$^3$ par 100 cm$^3$ de matériau poreux et les cellules à faire proliférer, dans lequel le remuage du matériau poreux est opéré par agitation, vibration, mélange ou une association de ces moyens.

## Patentansprüche

1. Zellkulturverfahren, umfassend einen Schritt der Bewegung von porösem Material, welches ein pro 100 cm$^3$ des porösen Materials imprägniertes flüssiges Medium in einer Menge von 15 cm$^3$ bis 60 cm$^3$ und die zu vermehrenden Zellen enthält, wobei die Bewegung des porösen Materials durch Schütteln, Vibration, Mischen oder eine Kombination dieser Methoden erfolgt.

# FIG. 1

# FIG. 2

# FIG. 3

# FIG. 4

EP 0 244 873 B1